(19) Europäisches Patentamt
European Patent Office
Office européen des brevets

(11) Veröffentlichungsnummer: **0 004 590**
**A1**

(12) # EUROPÄISCHE PATENTANMELDUNG

(21) Anmeldenummer: 79100826.1

(22) Anmeldetag: 19.03.79

(51) Int. Cl.²: C 07 D 501/20
C 07 D 499/64, A 61 K 31/545
A 61 K 31/43, A 23 K 1/17

(30) Priorität: 30.03.78 DE 2813771

(43) Veröffentlichungstag der Anmeldung: 17.10.79 Patentblatt 79/21

(84) Benannte Vertragsstaaten: BE CH DE FR GB IT NL

(71) Anmelder: **BAYER Aktiengesellschaft**
**Zentralbereich Patente,Marken und Lizenzen Bayerwerk**
**D-5090 Leverkusen 1(DE)**

(72) Erfinder: **König, Hans-Bodo, Dr.**
**Herberts Katernberg 10**
**D-5600 Wuppertal 1(DE)**

(72) Erfinder: **Metzger, Karl Georg**
**Pahlkestrasse 15**
**D-5600 Wuppertal 1(DE)**

(54) **Penicillin- und Cephalosporinderivate, Verfahren zu ihrer Herstellung sowie ihre Verwendung.**

(57) β-Lactam-Verbindungen der Penicillin- und Cephalosporin-Reihe der Formel

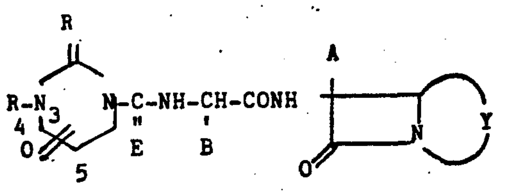

(I)

worin R einen Substituenten aber nicht Wasserstoff, E Sauerstoff oder Schwefel, B gegebenenfalls substituiertes Phenyl, gegebenenfalls substituiertes Heterocyclyl, Cyclohexenyl oder Cyclohexadienyl, u d A Wasserstoff, Hydroxy; Alkoxy, Alkenyloxy oder Cycloalkoxy bedeuten, und Verfahren zu ihrer Herstellung.

Diese Verbindungen zeigen neben guter antibakterieller Wirksamkeit eine ausgezeichnete Verträglichkeit und gute Löslichkeit, sowie die Eigenschaft, das Wachstum und die Futterverwertung bei Tieren zu verbessern.

EP 0 004 590 A1

Croydon Printing Company Ltd.

0004590



BAYER AKTIENGESELLSCHAFT 5090 Leverkusen, Bayerwerk
Zentralbereich
Patente, Marken und Lizenzen Jo-kl-bc

ß-Lactam-Verbindungen

BEZEICHNUNG GEÄNDERT
siehe Titelseite

Die vorliegende Erfindung betrifft neue ß-Lactam-Verbindungen, Verfahren zu ihrer Herstellung sowie ihre Verwendung.

Die neuen Verbindungen lassen sich durch Formel I wiedergeben

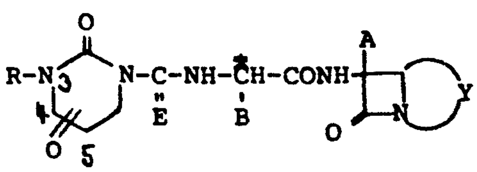

(I)

in der

R Alkyl, Alkenyl, Cycloalkyl, Cycloalkenyl, Cycloalkadienyl, wobei die genannten cyclischen Reste auch über eine Alkylengruppierung mit dem N-Atom verbunden sein können, Aralkyl, Aryl, Heterocyclyl, Heterocyclylalkyl, wobei die genannten Reste substituiert sein können oder eine Gruppe

$R_1$-CO- oder $R_2$-$SO_2$ ist,

in der

$R_1$, $R_2$ Alkyl, Alkenyl, Cycloalkyl, Cycloalkenyl, Cycloalkadienyl, wobei die genannten cyclischen Reste auch über eine Alkylengruppierung mit dem N-Atom verbunden sein können, Aralkyl, Aryl, Heterocylyl, Heterocyclylalkyl, wobei die für $R_1$, $R_2$ vorstehend genannten Reste substituiert sein können; Amino, $R_3$-NH-, $(R_3)_2$-N- oder $R_3$-CH=N- bedeutet, wobei

$R_3$ ein Substituent aus der Reihe Alkyl, Alkenyl, Cycloalkyl, Cycloalkylalkyl, Aralkyl, Aryl, Heterocyclyl, Heterocyclylalkyl ist und

$R_1$ zusätzlich Wasserstoff oder $R_3$-O- mit der oben angegebenen Bedeutung für $R_3$ bezeichnet, steht;

die zweite Carbonylgruppe in 4- oder 5-Stellung steht, mit der Bedingung, daß das 3-ständige N-Atom nicht 3-fach acyliert ist;

E für Sauerstoff oder Schwefel steht;

B gegebenenfalls substituiertes Phenyl, einen gegebenenfalls substituierten heterocyclischen Rest, Cyclohexenyl oder Cyclohexadienyl bezeichnet;

A Wasserstoff, -OH -O-$R_4$ bedeutet, wobei

$R_4$ Alkyl, Alkenyl oder Cycloalkyl ist, wobei die für $R_4$ genannten Reste substituiert sein können,

Y für die Gruppen

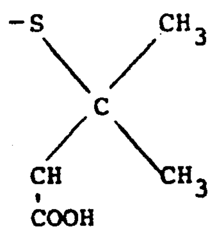

oder

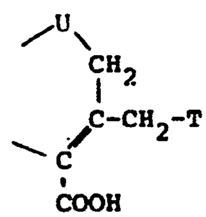

steht, in denen

U S, O, oder SO bezeichnet,

T Wasserstoff, Alkyl-CO-O-, Pyridinium, Aminopyridinium, Carbamoylpyridinium, Carbamoyloxy, Azido, Cyano, Hydroxy, die Gruppe -S-Phenyl, welche substituiert sein kann, oder die Gruppe -S-Het bedeutet, in welcher Het für einen gegebenenfalls substituierten heterocyclischen 5- oder 6-gliedrigen Ring steht;

und in welchen das Kohlenstoffatom, welches die Carboxylgruppe trägt, an das Stickstoffatom des ß-Lactamringes gebunden ist und

wobei diese Verbindungen der Formel I bezüglich des Chiralitätszentrums $\overset{*}{C}$ in den beiden möglichen R- und S-Konfigurationen sowie als Gemische der daraus resultierenden Diastereomeren vorliegen können, sowie die Salze und Ester der Verbindungen gemäß Formel I.

In Formel I steht als gegebenenfalls substituiertes Alkyl R, $R_1$, $R_2$, $R_3$ geradkettiges oder verzweigtes Alkyl mit vorzugsweise 1 bis 6, insbesondere 1 bis 4 Kohlenstoffatomen. Beipsielhaft seien gegebenenfalls substituiertes Methyl, Äthyl, n- und i-Propyl, n-, i- und t-Butyl genannt.

Als gegebenenfalls substituiertes Alkenyl R, $R_1$, $R_2$, $R_3$ steht geradkettiges oder verzweigtes Alkenyl mit vorzugsweise 2 bis 6, insbesondere 2 bis 4 Kohlenstoffatomen. Beispielhaft seien gegebenenfalls substituiertes Äthenyl, Propenyl-(1), Propenyl-(2) und Butenyl-(3), Butenyl-2- genannt.

Gegebenenfalls substituiertes Cycloalkyl, Cycloalkenyl und Cycloalkadienyl R, $R_1$, $R_2$, $R_3$ ist mono- oder bicyclisch und enthält vorzugsweise 3 bis 10, insbesondere 3, 5 oder 6 Kohlenstoffatome. Beispielhaft seien gegebenenfalls substituiertes Cyclopropyl, Cyclobutyl, Cyclopentyl, Cyclopentenyl, Cyclohexyl, Cyclohexenyl, Cyclohexadienyl, Cycloheptyl, Bicyclo-[2.2.1]-heptyl, Bicyclo-[2.2.2]-octyl und Adamantyl genannt.

Die genannten carbocyclischen Reste können auch über eine Alkylengruppierung mit 1 bis 4 C-Atomen, vorzugsweise eine $-CH_2-$ oder $-CH_2-CH_2-$Brücke mit dem N-Atom des 1,3-Diazacyclohexansystem verbunden sein.

Als gegebenenfalls substituiertes Aryl R, $R_1$, $R_2$, $R_3$ steht

Aryl mit vorzugsweise 6 bis 10 Kohlenstoffatomen im Arylteil. Beispielhaft seien gegebenenfalls substituiertes Phenyl oder Naphthyl genannt. Substituenten im Phenylring stehen in o-, m- oder p-Stellung. Weiterhin seien die Reste

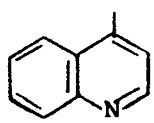 und 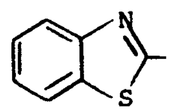

genannt.

Als gegebenenfalls substituiertes Aralkyl R, $R_1$, $R_2$, $R_3$ steht gegebenenfalls im Arylteil und/oder Alkylteil substituiertes Aralkyl mit vorzugsweise 6 oder 10, insbesondere 6 Kohlenstoffatomen im Arylteil und vorzugsweise 1 bis 4, insbesondere 1 oder 2 Kohlenstoffatomen im Alkylteil, wobei der Alkylteil geradkettig oder verzweigt sein kann. Beispielhaft seien gegebenenfalls substituiertes Benzyl und Phenyläthyl genannt.

Als gegebenenfalls substituiertes Heterocyclyl R, $R_1$, $R_2$, $R_3$ stehen heteroparaffinische, heteroaromatische und heteroolefinische 5- bis 7-gliedrige, vorzugsweise 5- oder 6-gliedrige Ringe mit vorzugsweise 1 bis 3, insbesondere 1 oder 2 gleichen oder verschiedenen Heteroatomen. Als Heteroatome stehen Sauerstoff, Schwefel oder Stickstoff. Als Beispiele seien gegebenenfalls substituiertes Thienyl, Furyl, Oxazolyl, Isoxazolyl, Thiazolyl, Isothiazolyl, Pyrrolyl, Imidazolyl, Pyrazolyl, Oxdiazolyl, Thiadiazolyl, Triazolyl, Oxtriazolyl, Thiatriazolyl, Tetrazolyl, Pyridyl, Pyrazinyl, Pyrimidinyl, Tetrahydrofuranyl, Dioxanyl, Pyrrolidinyl, Piperidinyl, Morpholinyl, Pyronyl-2 und -4.

Die genannten heterocyclischen Reste können auch über eine Alkylengruppierung mit 1 bis 4 C-Atomen, vorzugsweise eine $-CH_2-$ oder $-CH_2-CH_2-$Brücke mit dem N-Atom des 1,3-Diazacyclohexansystems verbunden sein.

Alkyl, Alkenyl, Cycloalkyl(alkyl), Cycloalkenyl(alkyl), Cycloalkadienyl(alkyl), Aralkyl, Aryl, R, $R_1$, $R_2$ und $R_3$ können einen oder mehrere vorzugsweise 1 bis 3, insbesondere 1 oder 2 gleiche oder verschiedene Reste $R_5$ tragen. Ganz besonders bevorzugt sind die genannten Reste R, $R_1$, $R_2$ und $R_3$ jedoch unsubstituiert oder tragen einen Substituenten $R_5$.

Heterocylyl(alkyl) R, $R_1$, $R_2$, $R_3$ kann einen oder mehrere, vorzugsweise 1 bis 3, insbesondere 1 oder 2 gleiche oder verschiedene Reste $R_6$ tragen. Ganz besonders bevorzugt ist Heterocylyl(alkyl) R, $R_1$, $R_2$, $R_3$ unsubstituiert oder enthält einen Substituenten $R_6$.

Bei den folgenden Darlegungen bedeutet der Ausdruck "Niederalkyl" überall, auch in Verbindung mit anderen Atomen oder Gruppen (z. B. Niederalkoxy, HCON-(Niederalkyl), usw.) geradkettiges oder verzweigtes Alkyl mit vorzugsweise 1 bis 6, insbesondere 1 bis 4 Kohlenstoffatomen. Beispielhaft seien gegebenenfalls substituiertes Methyl, Äthyl, n- und i-Propyl, n-, i- und t-Butyl genannt. "Niederalkyl" kann durch 1 bis 5, insbesondere 1 bis 3 gleiche oder verschiedene Halogenatome,

wobei als Halogenatome vorzugsweise Fluor, Chlor und Brom, insbesondere Fluor und Chlor stehen, substituiert sein. Beispielhaft seien Trifluormethyl, Chlor-di-fluormethyl, Brommethyl, 2,2,2-Tri-fluoräthyl und Pentafluoräthyl genannt.

$R_5$ bedeutet vorzugsweise Halogen, vorzugsweise Fluor, Chlor, Brom und Jod, insbesondere Fluor, Chlor und Brom; Amino; Mononiedrigalkylamino, vorzugsweise Methylamino, Äthylamino, insbesondere Methylamino; Diniedrigalkylamino, vorzugsweise Dimethylamino, Diäthylamino, insbesondere Dimethylamino; Pyrrolidyl; Piperidyl; HCO-NH-; Niederalkyl-CO-NH-, vorzugsweise $CH_3$-CO-NH-; H-CO-N(Niederalkyl)-, vorzugsweise H-CO-N($CH_3$)-, H-CO-N($C_2H_5$)-; Niederalkyl-CO-N(Niederalkyl)- vorzugsweise $CH_3$-CO-N($CH_3$)-;(Niederalkyl)$_2$C=N-; Niederalkyl-$SO_2$-NH-, vorzugsweise $CH_3$-$SO_2$-NH-, $C_2H_5$-$SO_2$-NH-, insbesondere $CH_3$-$SO_2$-NH-; Niederalkyl-$SO_2$-N(Niederalkyl)-, vorzugsweise $CH_3$-$SO_2$-N($CH_3$)-; HO-$SO_2$-NH-; HO-$SO_2$-N(Niederalkyl)-, vorzugsweise HO-$SO_2$-N($CH_3$)-, HO-$SO_2$-N($C_2H_5$)-; Amidino; (Niederalkyl)$_2$-N-CH=N-, insbesondere ($CH_3$)$_2$N-CH=N-;

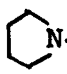N-CH=N-, Guanido, Nitro, Azido, Hydroxy, Niederalkyl-oxy-, vorzugsweise $CH_3$-O-, $C_2H_5$-O-, insbesondere $CH_3$O-, H-CO-O-, Niederalkyl-CO-O-, vorzugsweise $CH_3$-CO-O, $C_2H_5$-CO-O-, ($CH_3$)$_3$C-CO-O-; Niederalkyl-O-CO-O-, vorzugsweise $CH_3$-O-CO-O-, $C_2H_5$-O-CO-O-, ($CH_3$)$_3$C-O-CO-O-; $H_2$N-CO-O-; Niederalkyl-NH-CO-O-, vorzugsweise $CH_3$-NH-CO-O-, $C_2H_5$-NH-CO-O-; (Niederalkyl)$_2$N-CO-O-, vorzugsweise ($CH_3$)$_2$N-CO-O-; ($C_2H_5$)$_2$N-CO-O-, 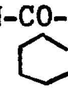N-CO-O-, $H_2$N-$SO_2$-O-; Niederalkyl-NH-$SO_2$-O-, vorzugsweise $CH_3$-NH-$SO_2$-O-, $C_2H_5$-NH-$SO_2$-O-, (Niederalkyl)$_2$-O-, vorzugsweise ($CH_3$)$_2$ N-$SO_2$-O-, ($C_2H_5$)$_2$ N-$SO_2$-O-; HOOC-, $H_2$N-CO-; (Niederalkyl)$_2$N-CO-, insbesondere ($CH_3$)$_2$N-CO- und ($C_2H_5$)$_2$N-CO-; OHC-, HO-$SO_2$-O-, HS-; Niederalkyl-S-,

vorzugsweise $CH_3$-S-,$CF_3$-S-, $C_2H_5$-S-, $(CH_3)_2$CH-S-; Niederalkyl-$\underset{O}{\underset{\Vert}{S}}$-, vorzugsweise $CH_3$-$\underset{O}{\underset{\Vert}{S}}$-, $C_2H_5$-$\underset{O}{\underset{\Vert}{S}}$-; $HO_3$S-;

Niederalkyl-$SO_2$-, vorzugsweise $CH_3$-$SO_2$-, $CF_3SO_2$-, $C_2H_5$-$SO_2$-; die Gruppe $H_2N$-$SO_2$-; Niederalkyl-NH-$SO_2$-, vorzugsweise $CH_3$-NH-$SO_2$-, $C_2H_5$-NH-$SO_2$-; (Niederalkyl)$_2$N-$SO_2$-, vorzugsweise $(CH_3)_2$ N-$SO_2$-, $(C_2H_5)_2$ N-$SO_2$-; 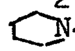-$SO_2$-; die Gruppe HO-$SO_2$-S-, geradkettiges oder verzweigtes Alkyl mit 1 bis 6 Kohlenstoffatomen, insbesondere Methyl, Äthyl, Propyl, Isopropyl, n-Butyl, sek.-Butyl oder tert.-Butyl, vorzugsweise Methyl; und Phenyl oder Phenoxy.

Im Falle, daß $R_6$ an einem oder mehreren Kohlenstoffatomen im Heterocyclyl R, $R_1$, $R_2$, $R_3$ steht, bedeutet $R_6$ vorzugsweise Niederalkyl, vorzugsweise Methyl, Äthyl, Isopropyl, insbesondere Methyl; die Gruppe Trifluormethyl; Halogen, vorzugsweise Fluor, Chlor, Brom; Amino; Niederalkylamino, vorzugsweise $CH_3$-NH-, $C_2H_5$-NH-; Diniederalkylamino, vorzugsweise $(CH_3)_2$N-, $(C_2H_5)_2$N-; Formylamino; Acetylamino; $CH_3$-O-CO-NH-, $C_2H_5$O-CO-NH-; $CH_3$-$SO_2$-NH-; Hydroxy; Methoxy, Äthoxy; Methylthio, Äthylthio; $CH_3$-$SO_2$-; $CH_3$-SO-; die Gruppen HOOC-; $HO_3$S-; HCO-; Niederalkyl-CO-, vorzugsweise $CH_3$-CO-; Niederalkyl-O-CO-, vorzugsweise $CH_3$-O-CO-, $C_2H_5$O-CO-; und -CN.

Im Falle, daß $R_6$ in einem stickstoffhaltigen Heterocylyl R, $R_1$, $R_2$, $R_3$ als Substituent an einem oder mehreren Stickstoffatomen steht, bedeutet $R^4$ vorzugsweise Niederalkyl, vorzugsweise Methyl, Äthyl, Propyl, Isopropyl, insbesondere Methyl und Äthyl; die Gruppe -C≡N; -CHO; -COO-Niederalkyl, vorzugs weise -COO-$CH_3$, -COO$C_2H_5$, -COOCH$(CH_3)_2$, -COO-C$(CH_3)_3$; -CO-$NH_2$; -CO-NH-Niederalkyl, vorzugsweise -CO-NH-$CH_3$, -CO-NH-$C_2H_5$,

$-CO-NH-CH(CH_3)_2$; und -CO-Niederalkyl, vorzugsweise $-CO-CH_3$, $-CO-C_2H_5$, $-CO-CH(CH_3)_2$.

$R_4$ bezeichnet einen durch Halogen, vorzugsweise Chlor und Brom, Amino, Monoalkylamino, Dialkylamino, wobei die Alkylsubstituenten am N-Atom der Aminogruppe je 1 bis 6 C-Atome enthalten können, oder Alkoxy mit 1 bis 6 C-Atomen substituierten Alkylrest; einen Alkenylrest mit 3 bis 6 C-Atomen, einen Alkinylrest mit 3 bis 6 C-Atomen, einen Cycloalkylrest mit 3 bis 7 C-Atomen einen Benzyl-, Phenyl- oder Naphthylrest.

Besonders bevorzugt sind jedoch die Verbindungen bei denen $R_4$ einen unsubstituierten Alkylrest mit 1 bis 4 C-Atomen wie Methyl, Äthyl, n-Propyl, i-Propyl, n-Butyl, i-Butyl oder $-CH_2CH_2N(CH_3)_2$ bezeichnet und ganz besonders solche, bei denen $R_4$ für Methoxy steht.

A steht jedoch bevorzugt für Wasserstoff und Methoxi und ganz besonders bevorzugt für Wasserstoff.

E steht bevorzugt für Sauerstoff.

Gegebenenfalls substituiertes Phenyl B kann einen oder mehrere, vorzugsweise 1 bis 3, insbesondere 1 oder 2 gleiche oder verschiedene Substituenten tragen. Die Substituenten können in o-, m- und/oder p-Stellung stehen. Vorzugsweise befindet sich ein Substituent in p- oder m-Stellung. Als Substituenten seien beispielhaft Halogen, wie Fluor, Chlor, Brom und Jod, vorzugsweise Fluor, Chlor und Brom; Alkyl mit 1 bis 6, vorzugsweise 1 bis 4, insbesondere 1 oder 2 Kohlenstoffatomen; Cyan und Methylsulfonyl aufgeführt. Insbesondere seien als substituierte Phenylreste B der Hydroxyphenylrest (vorzugsweise p-Hydroxyphenyl), der Methylphenylrest (vorzugsweise p-Methylphenyl), der Cyanphenylrest (vorzugsweise m- und p-Cyanphenyl), der Methylsulfonylrest (vorzugsweise p-Methylsulfonylphenyl) und der Fluorphenylrest (vorzugsweise o-Fluorphenyl und m-Fluorphenyl) genannt.

B in der Bedeutung eines gegebenenfalls substituierten heterocyclischen Restes steht vorzugsweise für einen fünfgliedrigen heterocyclischen Rest, der 1 oder 2 Heteroatome aus der Reihe N, O, S enthält und der durch 1 oder 2 Substituenten aus der Reihe Alkyl mit 1 bis 4 C-Atomen, vorzugsweise Methyl, Phenyl, Halogen, vorzugsweise Chlor und Brom, Amino substituiert sein kann. Als Beispiele seien aufgeführt:

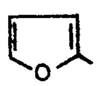 ; 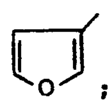 ; 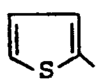 ; 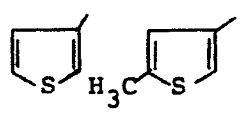 ;

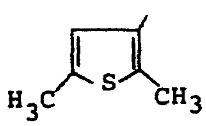 ; 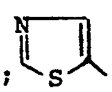 ; 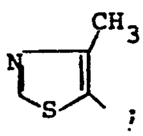 ; 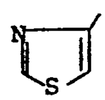 ; 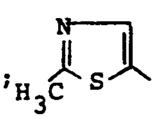 ;

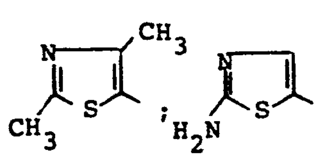 ; 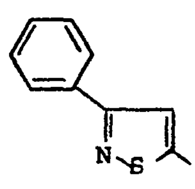 ; 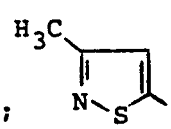 ; 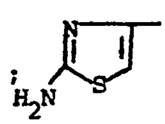 .

U steht besonders bevorzugt für S.

In der Definition von T bedeutet Alkyl in Alkyl-CO-O- vorzugsweise Alkyl mit 1 bis 4, insbesondere 1 oder 2 Kohlenstoffatome. Beispielhaft seien Methyl und Äthyl genannt, wobei Methyl besonders bevorzugt ist.

Der heterocyclische Ring Het in -S-Het (Definition von T) besteht aus 5 oder 6 Ringgliedern und enthält 1 bis 4, vorzugsweise 3 oder 4 gleiche oder verschiedene Heteroatome, wobei als Heteroatome Sauerstoff, Schwefel und Stickstoff stehen. Bevorzugt ist der heterocyclische Ring ungesättigt und enthält besonders bevorzugt 2 Doppelbindungen. Der heterocyclische Ring kann einen oder mehrere, vorzugsweise 1 oder 2, insbesondere einen Substituenten enthalten. Als Substituenten seien beispielhaft aufgeführt: Niederalkyl, Niederalkylamino, Diniederalkylamino, Diniederalkylaminoalkylen, Aminocarbonyl, Niederalkylaminocarbonyl, Diniederalkylaminocarbonyl, Carboxyalkylen, Sulfonylalkylen, Hydroxy, Acylamino, wobei der Acylrest bevorzugt Acetyl oder Formyl ist und wobei das Alkylen bevorzugt aus $-(CH_2)-_{1,2,3}$, besonders bevorzugt aus $-(CH_2)-_{1,2}$ besteht.

Als -S-Het seien als besonders bevorzugt aufgeführt:

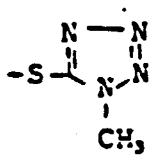 , 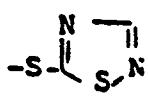 , 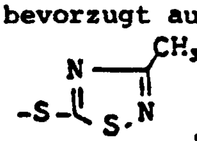 , 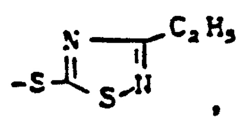 ,

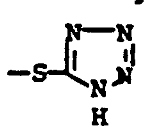 , 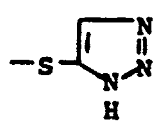 , 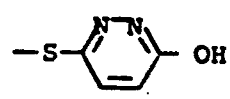 , 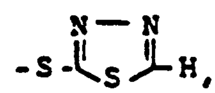 ,

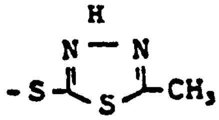 , 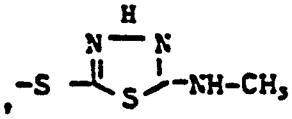 , 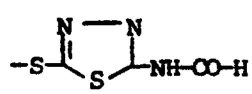 ,

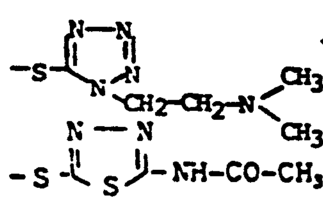 , 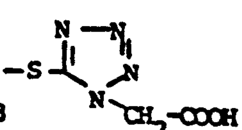 , 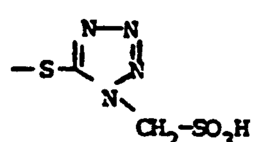 ,

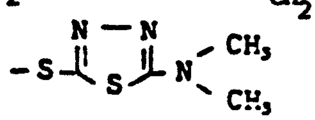 , 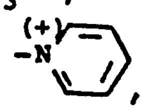 ,

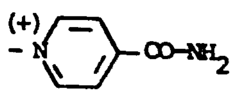 .

Der -S-Phenylrest in der Definition von T kann einen oder mehrere, vorzugsweise 1 bis 3, insbesondere 1 oder 2 gleiche oder verschiedene Substituenten tragen, wobei als Substituenten diejenigen bevorzugt werden, welche oben als mögliche Substituenten des Restes -S-Het aufgeführt werden.

Ganz besonders bevorzugte sind erfindungsgemäße Verbindungen, in welchen $\overset{*}{C}$ in der D-Konfiguration vorliegt.

Im Rahmen dieser Erfindung besitzen die pharmazeutisch verwendbaren Salze der Verbindungen gemäß Formel I besondere Bedeutung.

Pharmazeutisch verwendbare Salze der Verbindungen der Formel I sind Salze dieser Verbindungen mit anorganischen und organischen Basen an der sauren Carboxylgruppe bzw. den sauren Carboxyl- und Sulfonsäuregruppen. Als Basen können hierzu alle in der pharmazeutischen Chemie, insbesondere in der Chemie der Antibiotika, üblicherweise verwendeten Basen eingesetzt werden. Als anorganische Basen seien beispielhaft genannt: Alkali- und Erdalkalihydroxide, Alkali- und Erdalkalicarbonate und Alkalihydrogencarbonate, wie Natrium- und Kaliumhydroxid, Calcium- und Magnesiumhydroxid, Natrium- und Kaliumcarbonat, Calciumcarbonat, Natrium- und Kaliumhydrogencarbonat; Aluminiumhydroxid und Ammoniumhydroxid. Als organische Amine können primäre, sekundäre und tertiäre aliphatische Amine sowie heterocyclische Amine eingesetzt werden. Beispielhaft seien genannt: Di- und Triniedrigalkylamine, z.B. Diäthylamin, Triäthylamin, Tri-ß-hydroxyäthylamin, Procain, Dibenzylamin, N,N-Dibenzyläthylendiamin, N-Benzyl-ß-phenyläthylamin, N-Methyl- und N-Äthylmorpholin, 1-Ephenamin, Dehydroabietylamin, N,N'-Bis-Dehydroabietyläthylendiamin, N-Niedrigalkylpiperidin. Auch sogenannte basische Aminosäuren

wie Lysin oder Arginin können vorteilhaft als Basen Verwendung finden. Besonders bevorzugte Salze sind die Natriumsalze.

Für die bevorzugte Verwendung der erfindungsgemäßen Verbindungen auf dem Arzneimittelgebiet spielen fernerhin deren in vivo spaltbare Ester eine Rolle. Es handelt sich hierbei um Ester der Carboxylfunktion in 3-Stellung der Penam- bzw. 4-Stellung des Cephem-Gerüstes mit Alkoholen wie z.B. $HOCH_2OCH_3$, $HOCH_2OOCC(CH_3)_3$, $HOCH_2CH_2$-N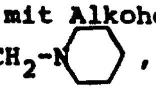,

$HOCH_2CH_2$-N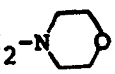 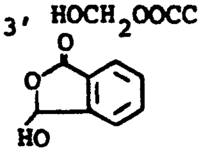

Im Rahmen der vorliegenden Erfindung kommt den ß-Lactam-Verbindungen gemäß Formel II

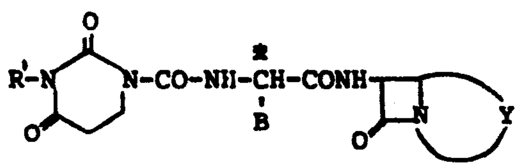 (II)

in der

R' für Alkyl mit 1 bis 6 Kohlenstoffatomen, Cycloalkyl mit 3 bis 6 Kohlenstoffatomen oder Phenyl

steht,

B für Phenyl, p-Hydroxyphenyl, Thienyl-(2), 1,4-Cyclohexadien-(1)-yl, 2-Aminothiazol-4-yl steht und

Y eine Gruppe

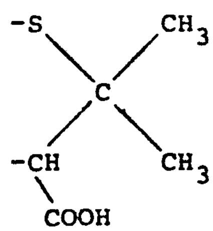

oder

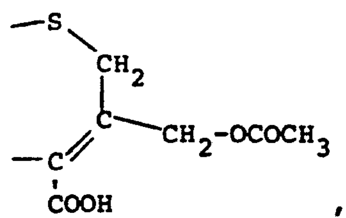

,

bei der das C-Atom, welches die Carboxylgruppe trägt, an das N-Atom des ß-Lactamringes gebunden ist,

und bei denen $\overset{*}{C}$ in der D-Konfiguration vorliegt, sowie deren Natriumsalzen, besondere Bedeutung zu.

Als Beispiele für erfindungsgemäße Verbindungen seien aufgeführt:

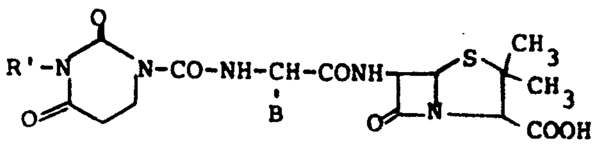

| R' | B |
| --- | --- |
| $CH_3-$ | 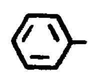 |
| $C_2H_5-$ | " |
| 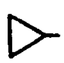 | " |
| 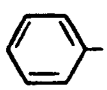 | " |
| 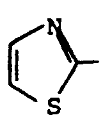 | " |
| 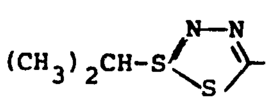 | " |

| R' | B |
|---|---|
| $CH_3$- | 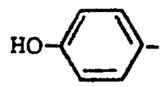 |
| $C_2H_5$- | " |
| 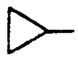 | " |
| 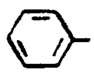 | " |
| 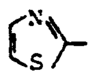 | " |
| 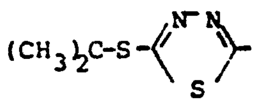 | " |
| H- | 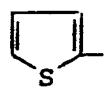 |
| $CH_3$- | " |
| $C_2H_5$- | " |
| 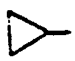 | " |
| 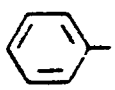 | " |
| 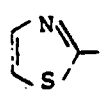 | " |
| 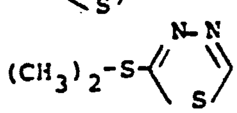 | " |

| R' | B |
| --- | --- |
| $CH_3-$ | 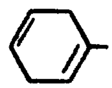 |
| $C_2H_5-$ | " |
| 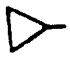 | " |
| 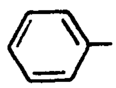 | " |
| 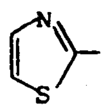 | " |
| $(CH_3)_2CH-S-$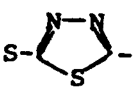 | " |
| $CH_3-$ | 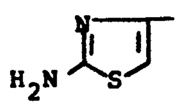 |
| $C_2H_5-$ | " |
| 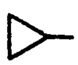 | " |
| 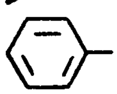 | " |
| 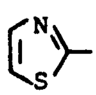 | " |
| 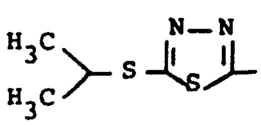 | " |

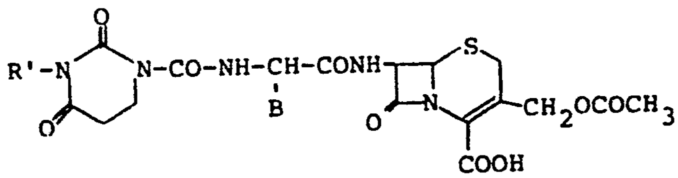

| R' | B |
|---|---|
| $CH_3$- | 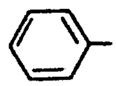 |
| $C_2H_5$- | " |
| 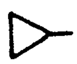 | " |
| 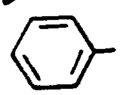 | " |
| 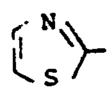 | " |
| $(CH_3)_2CH$-S-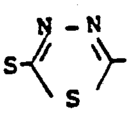 | " |
| $CH_3$- | HO-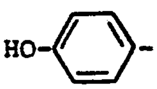 |
| $C_2H_5$- | " |
| 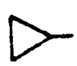 | " |
| 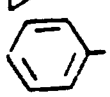 | " |
| 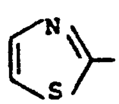 | " |
| $(CH_3)_2CH$-S-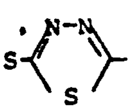 | " |

| R' | B |
|---|---|
| $CH_3-$ | 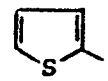 |
| $C_2H_5-$ | " |
| 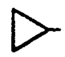 | " |
| 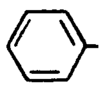 | " |
| 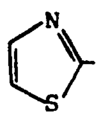 | " |
| 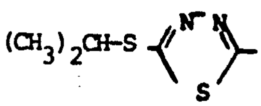 | " |
| $CH_3$ | 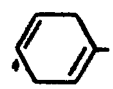 |
| $C_2H_5-$ | " |
| 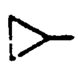 | " |
| 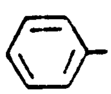 | " |
| 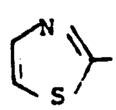 | " |
| 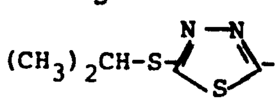 | " |

| R' | B |
|---|---|
| $CH_3$ | 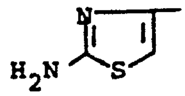 |
| $C_2H_5$- | " |
| 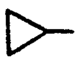 | " |
| 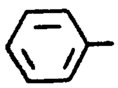 | " |
| 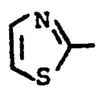 | " |
| 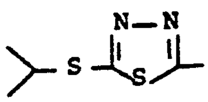 | " |

Als Beispiele für erfindungsgemäße Verbindungen seien aufgeführt:

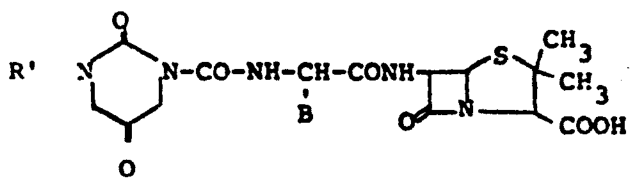

| R' | B |
|---|---|
| $CH_3$- | 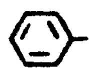 |
| $C_2H_5$- | " |
| 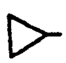 | " |
| 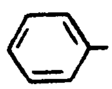 | " |
| 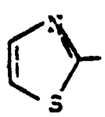 | " |
| $(CH_3)_2CH$-S 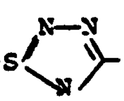 | " |
| 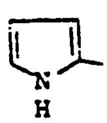 | " |

| R | B |
|---|---|
| 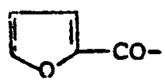 | 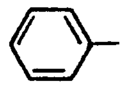 |
| $CH_3-SO_2-$ | " |
| 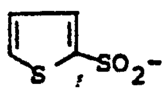 | " |
| 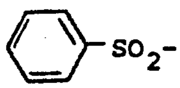 | " |
| $CH_3-$ | 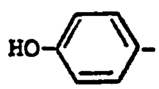 |
| $C_2H_5-$ | " |
| 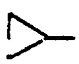 | " |
| 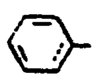 | " |
| 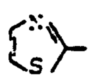 | " |
| $(CH_3)_2-S-$ 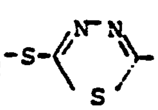 | " |
| 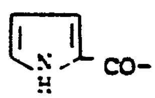 | " |
| 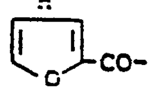 | " |

| | B |
|---|---|
| $CH_3-SO_2-$ | 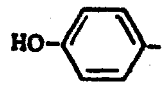 |
| 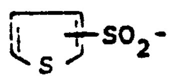 | " |
| 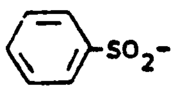 | " |
| H- | 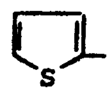 |
| $CH_3-$ | " |
| $C_2H_5-$ | " |
| 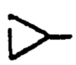 | " |
| 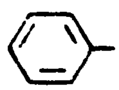 | " |
| 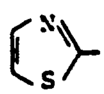 | " |
| $(CH_3)_2-S-$ 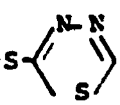 | " |
| 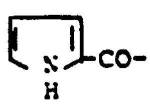 | " |
| 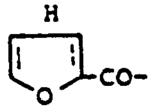 | " |
| $CH_3-SO_2-$ | " |

0004590



| R | B |
|---|---|
| 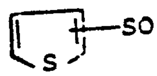 $-SO_2-$ | 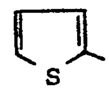 |
| 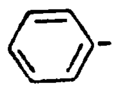 $-SO_2-$ | " |
| $CH_3-$ | 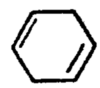 |
| $C_2H_5-$ | " |
| 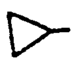 | " |
| 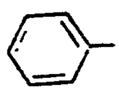 | " |
| 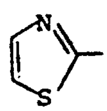 | " |
| $(CH_3)_2CH-S-$ 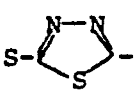 | " |
| 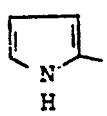 CO- | " |
| 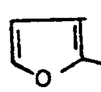 CO- | " |

| R | B |
|---|---|
| $CH_3SO_2-$ | 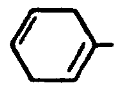 |
| 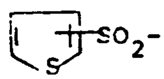 | " |
| 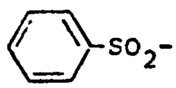 | " |
| $CH_3$ | 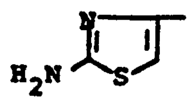 |
| $C_2H_5-$ | " |
| 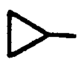 | " |
| 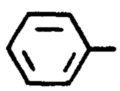 | " |
| 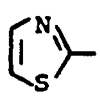 | " |
| 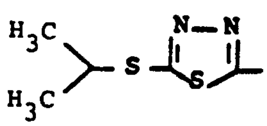 | " |
| 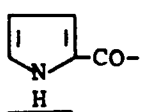 | " |
| 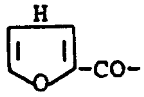 | " |
| $CH_3-SO_2-$ | " |
| 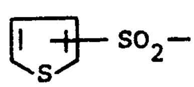 | " |
| 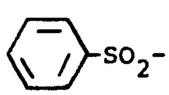 | " |

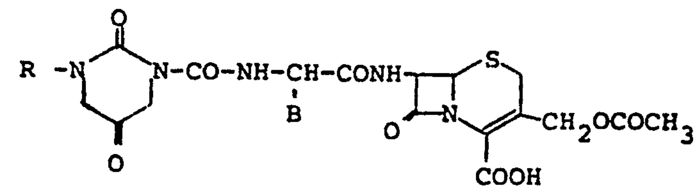

| R' | B |
|---|---|
| $CH_3$- | 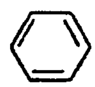 |
| $C_2H_5$- | " |
| 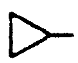 | " |
| 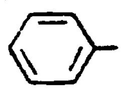 | " |
| 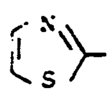 | " |
| 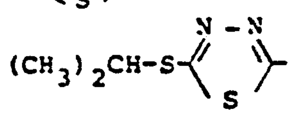 | " |
| 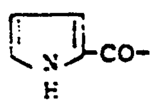 | " |
| 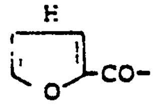 | " |
| $CH_3$-$SO_2$- | " |
| 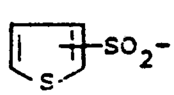 | " |
| 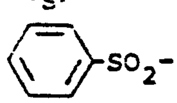 | " |

| R' | B |
|---|---|
| $CH_3-$ | 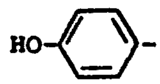 |
| $C_2H_5-$ | " |
| 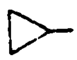 | " |
| 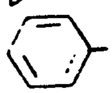 | " |
| 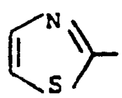 | " |
| $(CH_3)_2CH-S-$ 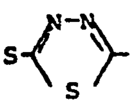 | " |
| 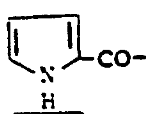 | " |
| 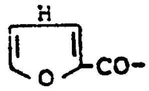 | " |
| $CH_3-SO_2-$ | " |
| 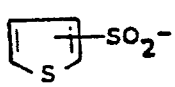 | " |
| 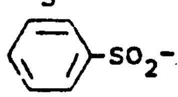 | " |

| R' | B |
|---|---|
| $CH_3-$ | 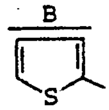 |
| $C_2H_5-$ | " |
| 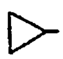 | " |
| 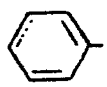 | " |
| 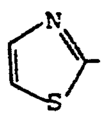 | " |
| (CH$_3$CH-S- 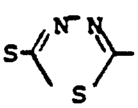 | " |
| 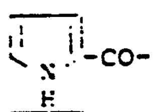 | " |
| 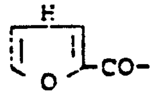 | " |
| $CH_3-SO_2-$ | " |
| 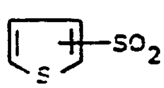 | " |
| 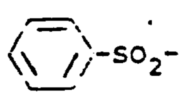 | " |

| R' | B |
|---|---|
| $CH_3$ | 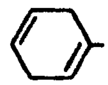 |
| $C_2H_5$- | " |
| 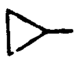 | " |
| 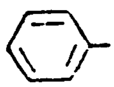 | " |
| 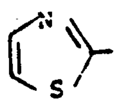 | " |
| $(CH_3)_2CH$-S-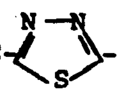 | " |
| 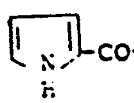CO- | " |
| 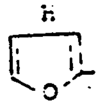CO- | " |
| $CH_3$-$SO_2$- | " |
| 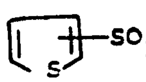-$SO_2$- | " |
| 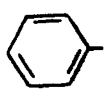-$SO_2$- | " |
| $CH_3$ | 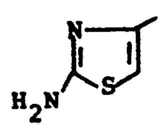 |
| 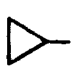 | " |

| R' | B |
| --- | --- |
| 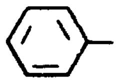 | 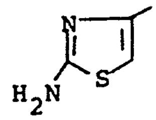 |
| 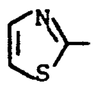 | " |
| $CH_3SO_2-$ | " |
| 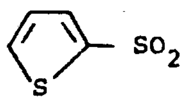 | " |

Die erfindungsgemäßen Verbindungen zeigen neben guter antibakterieller Wirksamkeit eine ausgezeichnete Verträglichkeit und gute Löslichkeit, so daß sie in vorteilhafter Weise in der Human- und Veterinärmedizin Verwendung finden können.

Daneben zeigen sie die Eigenschaft, das Wachstum und die Futterverwertung bei Tieren zu verbessern.

Überraschenderweise sind sie in Wirkungsbreite, Wirkungsintensität und/oder Verträglichkeit den strukturell nächststehenden Verbindungen des Standes der Technik (siehe DT-OS 2 025 414, DT-OS 2 025 415, DT-OS 2 104 580, DT-OS 2 104 579, DT-OS 2 152 967, DT-OS 2 152 968, DT-OS 2 519 400, DT-OS 2 258 973) überlegen. Die erfindungsgemäßen Verbindungen stellen somit eine Bereicherung und Ergänzung des Arzneimittelschatzes dar.

Weiterhin wurde gefunden, daß man die neuen ß-Lactam-Verbindungen gemäß Formel I erhält, wenn man Verbindungen der Formel III

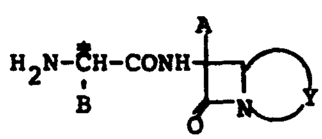

(III)

in der A, B, Y und $\overset{*}{C}$ die oben angegebene Bedeutung besitzen,

mit Verbindungen der Formel (IV)

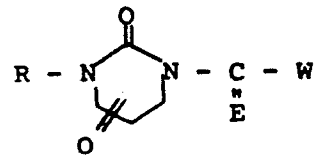 (IV)

oder wenn man Verbindungen der Formel V

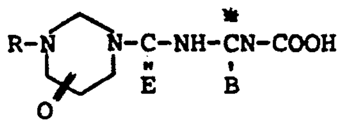

in einer an der Carboxylgruppe aktivierten Form, mit Verbindungen der Formel VI

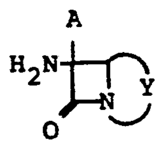

in der

R und E die oben angegebene Bedeutung haben und

W für Halogen, insbesondere Cl, Azid oder eine andere nukleofuge Abgangsgruppe steht,

in Gegenwart eines Lösungsmittels und gegebenenfalls eines Säurebindemittels bei Temperaturen von etwa -20 bis etwa +50°C umsetzt, gegebenenfalls Aminoschutzgruppen entfernt und die erhaltenen ß-Lactam-Antibiotica gegebenenfalls in Salze und Ester überführt oder aus den erhaltenen Salzen gewünschtenfalls die freien Säuren herstellt.

Ferner können die neuen ß-Lactam-Verbindungen der Formel I die anstelle von A den Rest $-OR_2$ tragen, dadurch herstellen, daß man die Verbindungen der Formel I, die anstelle von A Wasserstoff tragen und in denen R, $R_2$, E, B und Y die oben angegebene Bedeutung haben, mit 2 bis 10 Äquivalenten einer Base in Anwesenheit eines Überschusses eines Alkohols der Formel $R_2OH$, in der $R_2$ die oben angegebene Bedeutung besitzt, in einem inerten organischen Lösungsmittel umsetzt, zwischen 1 bis 8 Äquivalente eines N-Halogenierungsmittels zufügt und die Verbindung der Formel (I), gegebenenfalls nach vorheriger Abspaltung der Säureschutzgruppe und/oder Überführung in ein Salz, isoliert.

Verwendet man beispielsweise D-α-Amino-benzylpenicillin und 1-Chlorcarbonyl-3-methyl-2,4-dioxo-1,3-diazacyclohexan als Ausgangsstoffe, so kann ein Reaktionsablauf durch das folgende Formelschema wiedergegeben werden:

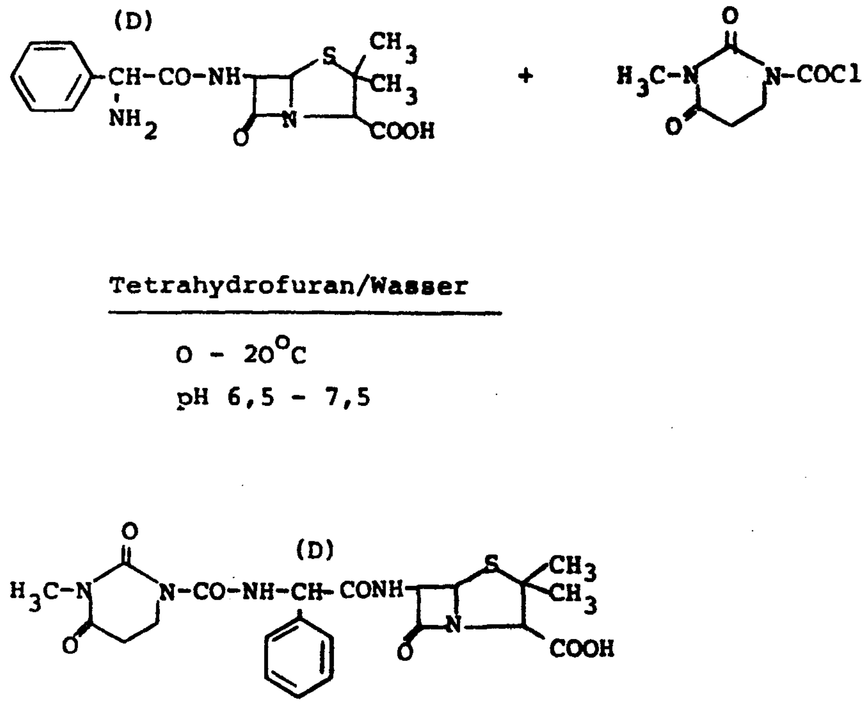


Die als Ausgangsstoffe verwendeten Verbindungen der allgemeinen Formel III sind bereits bekannt oder nach bekannten Methoden erhältlich.

Alle Kristallformen, Hydratformen und Salze der Verbindungen der allgemeinen Formel III sind als Ausgangsmaterialien für das erfindungsgemäße Verfahren geeignet.

Als Beispiele seien genannt:

α-Aminobenzylpenicillin, α-Amino-p-hydroxybenzylpenicillin, α-Amino-p-methylbenzylpenicillin, α-Amino-p-chlorbenzyl-

penicillin, 6-/2-Amino-2-(1,4-cyclohexadien-1-yl)-acetamido7-penicillansäure, 7-(α-Amino-phenylacetamido)-3-methyl-ceph-3-em-4-carbonsäure, 7-(α-Amino-phenylacetamido)-3-acetoxymethyl-ceph-3-em-4-carbonsäure, 7-(2-Amino-phenylacetamido)-7-methoxy-3-methyl-ceph-3-em-4-carbonsäure, 7-(2-Amino-phenylacetamido)-7-methoxy-3-acetoxymethyl-ceph-3-em-4-carbonsäure oder das entsprechende trifluoressigsaure Salz.

Als Salze der Verbindungen der Formel III können vorzugsweise Salze mit Basen eingesetzt werden, welche als für die Salzbildung mit Verbindungen der Formel I geeignet aufgeführt werden. Besonders bevorzugt sind die Natriumsalze.

Die als Ausgangsstoffe verwendeten Verbindungen der allgemeinen Formel IV sind bekannt oder nach bekannten Methoden erhältlich. Sie können z.B. auf folgendem Wege erhalten werden (vg. A. L. J. Beckwith et al.: J. Chem. Soc. C 1968, 2759):

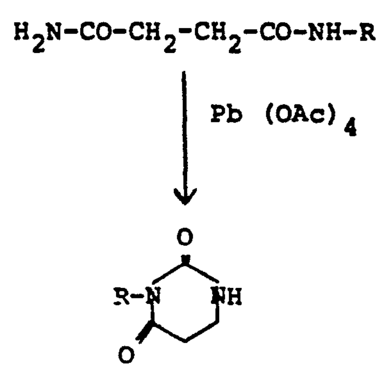

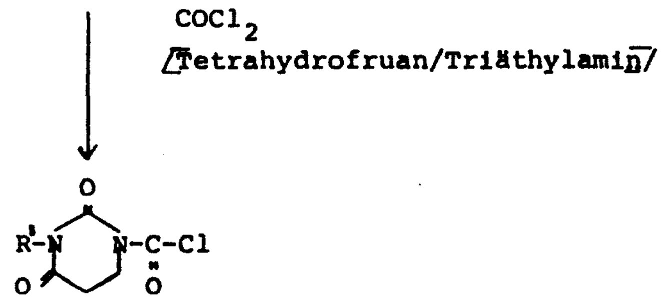


Als Verdünnungsmittel kommen beim erfindungsgemäßen Verfahren Wasser sowie alle inerten organischen Lösungsmittel, vorzugsweise solche, welche mit Wasser mischbar sind, in Frage. Hierzu gehören vor allem niedere Dialkylketone, z.B. Aceton, Methyläthylketon, cyclische Äther, z.B. Tetrahydrofuran und Dioxan; Nitrile, z.B. Acetonitril; niedere Dialkylformamide, z.B. Dimethylformamid; niedere Alkylalkohole, z.B. Äthanol und Isopropanol sowie Dimethylsulfoxid. Diese Lösungsmittel können auch in Mischungen untereinander sowie in beliebigen Mischungen einzelner oder mehrerer dieser Lösungsmittel mit Wasser verwendet werden. Das erfindungsgemäße Verfahren kann also durchgeführt werden in Gegenwart von: (a) ausschließlich Wasser, (b) ausschließlich einem oder mehreren organischen Lösungsmitteln oder (c) Wasser und einem oder mehreren organischen Lösungsmitteln. Ist wegen des Vorhandenseins von Wasser eine pH-Messung während der erfindungsgemäßen Reaktion möglich, wird der pH der Reaktionsmischung durch Zusatz von Basen oder durch Verwendung von Puffergemischen vorzugsweise zwischen 6,5 und 7,5 gehalten. Das erfindungsgemäße Verfahren läßt sich aber auch sehr gut in einem anderen pH-Bereich, beispielsweise zwischen 4,5 und 9,0 oder bei pH 2,0 bis 4,5, durchführen. Ferner ist

es möglich, die Reaktion in mit Wasser nicht mischbaren Lösungsmitteln, z.B. halogenierten Kohlenwasserstoffen, wie Chloroform oder Methylenchlorid, unter Zusatz von organischen Basen, vorzugsweise Niederalkylaminen, z.B. Triäthylaminen, Diäthylaminen oder cyclischen Basen, z.B. N-Äthylpiperidin durchzuführen. Weiterhin läßt sich die Reaktion in einer Mischung aus Wasser und einem mit Wasser nicht mischbaren Lösungsmittel, wie z.B. Niederalkyläthern, wie Diäthyläther, halogenierten Kohlenwasserstoffen, wie Chloroform und Methylenchlorid; Schwefelkohlenstoff; Isobutylmethylketon; Estern wie Essigsäureäthylester; aromatischen Kohlenwasserstoffen wie Benzol, ausführen, wobei es zweckmäßig ist, kräftig zu rühren und den pH-Wert durch Basenzusatz oder Verwendung von üblichen Pufferlösungen, z.B. Phosphat-, Acetat- oder Citratpuffer, zwischen 4,5 und 9,0 oder z.B. 2,0 und 4,5 zu halten. Man kann die Reaktion aber auch in Wasser allein in Abwesenheit von organischen Lösungsmitteln in Gegenwart einer organischen oder anorganischen Base oder unter Zusatz von üblichen Pufferstoffen durchführen.

Als Säurebindemittel können alle in der Chemie der Antibiotica üblicherweise verwendeten Säurebinder verwendet werden. Hierzu gehören anorganische Basen und organische Basen, welche z.B. durch sterische Hinderung schwer acylierbar sind. Als Beispiele für anorganische Basen seien Natrium- und Kaliumhydroxid genannt. Als orgaNische Basen kommen praktisch alle nicht oder schwer acylierbaren offenkettigen oder cyclischen Amine und auch heteroaromatische Basen in Frage. Als Basen seien beispielhaft tertiäre Amine, vorzugsweise Niederalkylamine, z.B. Triäthylamin und/oder cyclische Basen, z.B. Pyridin sowie als schwer acylierbares sekundäres Amin Dicyclohexylamin genannt.

Beim erfindungsgemäßen Verfahren ist der Zusatz einer Base nur dann erforderlich, wenn während der Reaktion saure Verbindungen entstehen, z.B. im Falle, daß W für Halogen oder Azid steht.

Die Reaktionstemperaturen können in einem größeren Bereich variiert werden. Im allgemeinen arbeitet man zwischen etwa -20°C und etwa +50°C, vorzugsweise zwischen 0 und +20°C. Wie bei den meisten chemischen Reaktionen können jedoch prinzipiell auch höhere oder niedrigere Temperaturen verwendet werden.

Die Umsetzung kann bei Normaldruck, aber auch bei vermindertem oder erhöhten Druck ausgeführt werden. Im allgemeinen arbeitet man bei Normaldruck.

Bei der Durchführung der erfindungsgemäßen Verfahren können die Anteile der Reaktionspartner in den Formeln III und IV in weiten Grenzen variiert werden, ohne daß das Ergebnis nachteilig beeinflußt wird. Die Ausgangstoffe können z.B. in äquimolaren Mengen miteinander zur Reaktion gebracht werden. Es kann jedoch zweckmäßig sein, einen der beiden Reaktionspartner im Überschuß zu verwenden, um sich die Reinigung oder Reindarstellung der gewünschten ß-Lactam-Verbindung zu erleichtern und die Ausbeute zu erhöhen.

Beispielsweise kann man die Reaktionspartner der allgemeinen Formel III mit einem Überschuß von 0,1 bis 0,3 Moläquivalenten einsetzen und dadurch eine geringere Zersetzung der Reaktionspartner der allgemeinen Formel IV in einem

wasserhaltigen Lösungsmittelgemisch erreichen. Der Überschuß der Reaktionspartner der allgemeinen Formel III läßt sich wegen der guten Löslichkeit in wäßrigen Mineralsäuren beim Aufarbeiten des Reaktionsgemisches leicht entfernen.

Andererseits kann man aber auch mit Vorteil der Reaktionspartner der allgemeinen Formel IV mit einem Überschuß von beispielsweise 0,1 bis 1,0 Moläquivalenten einsetzen. Dadurch werden die Reaktionspartner der allgemeinen Formel III besser ausgenützt und die als Nebenreaktion in wasserhaltigen Lösungsmitteln ablaufende Zersetzung der Reaktionsteilnehmer der allgemeinen Formel IV kompensiert.

Die Menge der gegebenenfalls verwendeten Basen ist z.B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich ist oder nicht sinnvoll ist, werden vorzugsweise 2 Moläquivalente Base zugesetzt.

Die Aufarbeitung der Reaktionsansätze zur Herstellung der erfindungsgemäßen Verbindungen und ihrer Salze erfolgt durchweg in der bei diesen Körpern allgemein bekannten Art und Weise. Auch die Isolierung und Reinigung der erfindungsgemäßen Verbindungen sowie die Freisetzung der freien Säuren aus Salzen oder die Umwandlung der freien Säuren in Salze werden nach allgemein üblichen Methoden der organischen Chemie, welche jedem Fachmann geläufig sind, vorgenommen.

Die Verbindungen der allgemeinen Formel I sind in Form der freien Säure sowohl kristallin wie amorph und sowohl wasserfrei wie in verschiedenen Hydratformen in gleicher Weise antibakteriell wirksam. Ebenfalls sind die Verbindungen der allgemeinen Formel I in Form ihrer Salze, z.B. Natriumsalze sowohl kristallin wie amorph und sowohl wasserfrei wie wasserhaltig, beispielsweise als Hydrat, in gleicher Weise antibakteriell wirksam.

Die erfindungsgemäßen Wirkstoffe weisen bei guter Verträglichkeit eine starke und breite antimikrobielle Wirksamkeit auf. Diese Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Wirkstoffe sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Wirkstoffe gegen Bakterien und bakterienähnliche Mikroorganismen. Sie sind daher besonders gut zur Prophylaxe und Chemotherapie von lokalen und systemischen Infektionen in der

Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph.epidermidis;

Lactobacteriaceae, wie Streptokokken, z.B. Streptococcus pyogenes, α- bzw. β-hämolysierende Streptokokken, nicht (γ-)-hämolysierende Streptokokken, Str.viridans, Str. faecalis (Enterokokken), und Diplococcus pneumoniae (Pneumokokken) (Str. = Streptococcus);
Neisseriaceae, wie Neisserien, z.B. Neisseria gonorrhoeae (Gonokokken), N.meningitidis (Meningokokken), N.catarrhalis (N. = Neisseria);
Corynebacteriaceae, wie Corynebakterien, z.B. Corynebacterium diphtheriae, C. pyogenes;

Enterobacteriaceae, wie Escherichiae-Bakterien der Coli-Gruppe: Escherichia-Bakterien, z.B. Escherichia coli, Enterobacter-Bakterien, z.B. E.aerogenes, E.cloacae, Klebsielle-Bakterien, z.B. K.pneumoniae, Serratia, z.B. Serratia marcescens (E. = Enterobacter) (K. = Klebsiella), Proteae-Bakterien der Proteus-Gruppe: Proteus, z.B. Proteus vulgaris, Pr.morganii, Pr.rettgeri, Pr.mirabilis, Providencia, z.B. Providencia sp. (Pr. = Proteus), Salmonelleae: Salmonella-Bakterien, z.B. Salmonella paratyphi A und B, S.typhi, S.enteritidis, S.typhimurium

(S. = Salmonella), Shigella-Bakterien, z.B. Shigella dysenteriae, Sh.flexneri (Sh. = Shigella);

Pseudomonadaceae, wie Pseudomonas-Bakterien, z.B. Pseudomonas aeruginosa, (Ps. = Pseudomonas);

Bacteroidaceae, wie Bacteroides-Bakterien, z.B. Bacteroides fragilis;

Bacillaceae, wie aerobe Sporenbildner, z.B. Bacillus anthracis (B.subtilis, B.cereus) (B. = Bacillus), anaerobe Sporenbildner-Chlostridien, z.B. Clostridium perfringens, Cl. tetani, Cl.botulinum (Cl. = Clostridium).

Die obige Aufzählung von Erregern ist lediglich beispielhaft und keineswegs beschränkend aufzufassen.

Als Krankheiten, die durch die erfindungsgemäßen Wirkstoffe verhindert, gebessert und/oder geheilt werden können, seien beispielsweise genannt:

Erkrankungen der Atmungswege und des Rachenraumes;

Otitis; Pharyngitis; Pneumonie; Peritonitis; Pyelonephritis; Cystitis; Endocarditis; Systeminfektionen; Bronchitis; Arthritis; lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich

einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein

und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykol, Fette, z. B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Pufer und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensobit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterninärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Stoffe in Gesamtmengen von etwa 5 bis 1000, vorzugsweise 20 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 10 bis 100 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalt welchem die Verabreichung erfolgt. So kann es in einigen Fällen ausreichend sein, mit weniger als der obengenannten Menge Wirkstoff auszukommen, während in anderen Fällen die oben angeführten Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die neuen Verbindungen zeichnen sich durch starke antibakterielle Wirkungen, die in vivo und in vitro geprüft wurden, und durch orale Resobierbarkeit aus.

Die erfindungsgemäßen ß-Lactam-Verbindungen können zum Zwecke der Erweiterung des Wirkungsspektrums und um eine Wirkungssteigerung speziell bei ß-lactamasebildenden Bakterien zu

erzielen mit anderen antimikrobiellen Wirkstoffen z.B. mit Penicillinen, die besonders penicillinasefest sind, kombiniert werden. Eine Solche Kombination wäre z.B. die mit Oxacillin oder Dicloxacillin.

Die erfindungsgemäßen ß-Lactam-Verbindungen können zum Zweck der Erweiterung des Wirkungsspektrums und um eine Wirkungssteigerung zu erreichen auch mit Aminoglykosidantibiotica, wie z.B. Gentamicin, Kanamicin, Amikacin, Sisomicin oder Tobramicin, kombiniert werden.

In vivo-Versuche

Aus der folgenden Tabelle 1 geht die Wirkung einer der erfindungsgemäßen Verbindungen gegen eine Reihe von Bakterien im Tierversuch mit der weißen Maus hervor. Die weißen Mäuse vom Stamm $CF_1$ wurden intraperitoneal mit der jeweils angegebenen Bakterienart infiziert.

Tabelle 1

Tierversuch mit der weißen Maus

Bestimmung der $ED_{50}$ nach 24 Stunden

| Keim | Dosis in mg des ß-Lactamantibioticums des Beispiels 9 pro kg/Körpergewicht (subcutan) |
|---|---|
| Escherichia coli Neumann | 1 x 300 |

Therapie: zweimalig : 30 und 90 Minuten nach der Infektion. Die $ED_{50}$ ist die Dosis, bei der 50 % der infizierten Tiere nach 24 Stunden nocht überleben.

Das erfindungsgemäße Verfahren sei durch die folgenden Beispiele erläutert:

Bei den NMR-Spektrem der erfindungsgemäßen Verbindungen bedeuten die Bezeichnungen in den Klammern:

s = Singulett
d = Dublett
dd = Doppeldublett
$A_2B_2$ = $A_2B_2$-System

Erläuterungen der in den Beispielen verwendeten Abkürzungen:

Gew.-Tle. = Gewichtsteile
Vol.-Tle. = Volumenteile
THF = Tetrahydrofuran
Essigester = Essigsäureäthylester
Zers.-p = Zersetzungspunkt

Die Ausbeuteangaben in % bedeuten Ausbeuten in % der Theorie.

Volumen- und Gewichtsteile verhalten sich zueinander wie ml und g.

Beispiel 1

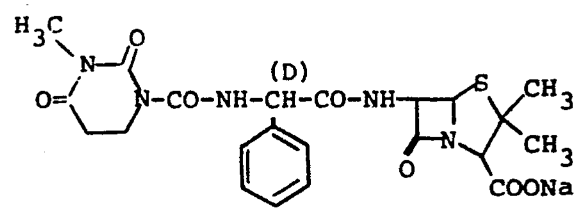

α-Aminobenzylpencillin (Ampicillin, Trihydrat) (2,0 Gew.-Teile) werden in 80 %igem wäßrigen Tetrahydrofuran (50 Vol.-Teile) suspendiert und Triäthylamin zugegeben, bis das Penicillin gerade eben gelöst ist. Dann wird eine Lösung von 1-Chlorcarbonyl-3-methyl-2,4-dioxo-1,3-diazacyclohexan (0,9 Gew.-Teile, bezogen auf Reinsubstanz) in etwas Tetrahydrofuran langsam unter Rühren und Kühlung mit Eis/Wasser zugegeben und dabei der pH der Mischung durch entsprechendes Zugeben von Triäthylamin auf 7,5 gehalten. Wenn kein Triäthylamin zur Aufrechterhaltung des pH-Wertes von 7,5 mehr zugegeben werden muß, wird die Mischung mit 100 ml Wasser verdünnt, einmal mit Ähter ausgeschüttelt, die wäßrige Phase nach Überschichten mit Essigester Rühren und unter Kühlung mit Eis/Wasser bis auf pH 2 mit verdünnter HCl angesäuert, die organische Phase abgetrennt, die reine wäßrige Phase nochmals mit Essigester ausgeschüttelt und die vereinigten Essigester-Phasen über Natriumsulfat getrocknet. Diese Lösung wird dann mit Äther auf das doppelte Volumen verdünnt und durch Zugabe einer etwa Methanol enthaltenden Natrium-2-äthylhexanorat-Lösung in Äther als Natriumsalz des Penicillins ausgefällt. Die Fällung war zunächst klebrig,

konnte jedoch durch Behandlung mit Äther der 10 % Methanol enthielt in ein lockeres farbloses Pulver überführt werden.

Ausbeute: 0,8 Gew.-Teile
Schmp.: ab ca. 200°C Zersetzung

$C_{22}H_{24}N_5NaO_7S$ x 1,5 $H_2O$

ber. C 47,8 H 4,9 N 12,7 S 5,8
gef. C 47,8 H 5,9 N 12,8 S 5,8

NMR (ppm, $CD_3OD$, 60 MHz): 1,48 (3 H, s, $\times^{CH_3}_{CH_3}$), 1,55 (3 H, s, $\times^{CH_3}_{CH_3}$), 2,68 (2 H, t, $-CH_2-CO-N-CH_3$), 3,13 (3 H, s, $N-CH_3$), 3,91 (2 H, t, $-CH_2-N<^{CO}_{CO}$), 4,10 (1 H, s, 3-H), 5,40 (2 H, AB, 5,6-H), 5,53 (1 H, s, $-\underset{N}{CH}-CO$), 7,55-7,08 (5 H, m, Phenyl).

Wirksamkeit (NHK in E/ml) bei E. coli Neumann = 4

Beispiel 2

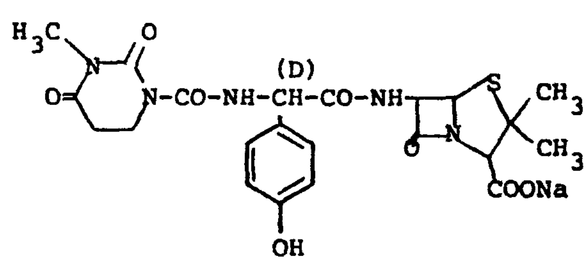

Dieses Penicillin wird in der in Beispiel 1 beschriebenen Weise aus 2,2 Gew.-Teilen α-Amino-p-hydroxybenzylpenicillin

(Amoxycillin, Trihydrat) und 1,5 Gew.-Teilen (bezogen auf Reinsubstanz) 1-Chlorcarbonyl-3-methyl-2,4-dioxo-1,3-diazacyclohexan hergestellt.

Ausbeute: 1,2 Gew.-Teile
Schmp. ca. 200°C Zersetzung

Die Substanz enthält nach dem NMR-Spektrum 0,36 Moläquivalente Natrium-2-äthylhexanoat und 2 Moläquivalente Wasser. Dieses wurde bei den berechneten Analysendaten berücksichtigt.

| | | | | | |
|---|---|---|---|---|---|
| Analse: | ber. | C 46,9 | H 5,2 | N 11,0 | S 5,0 |
| | gef. | C 46,9 | H 5,3 | N 11,0 | S 5,1 |

NMR (ppm $CD_3OD$, 60 MHz): 1,51 (3 H, s, $\times^{CH_3}_{CH_3}$), 1,58 (3 H, s, $\times^{CH_3}_{CH_3}$), 2,70 (2 H, t, $-CH_2-CO-\overset{|}{N}-CH_3$), 3,13 (3 H, s, $N-CH_3$), 3,91 (2 H, t, $-CH_2-N<^{CO}_{CO}$), 4,11 (1 H, s, 3-H), 5,29 - 5,54 (3 H, m, $-\underset{N}{CH}-CO$ u. 5,6-H), 6,96 (4 H, AB, HO-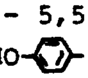-).

Wirksamkeit (MHK in E/ml) bei Serratia 16 001 = 16

<u>Beispiel 3</u>

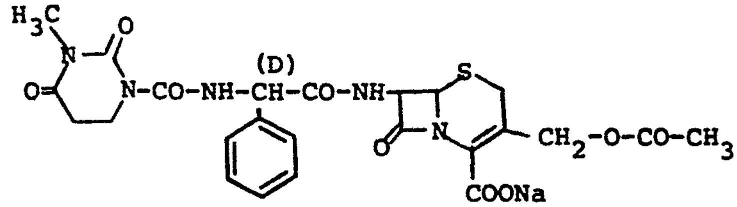

Dieses Cephalosporin wird in der im Beispiel 1 beschriebenen Weise aus 2,5 Gew.-Teilen α-Aminobenzyl-cephalosporin (Cephaloglycin, Dihydrat) und 1,0 Gew.-Teilen (bezogen auf Reinsubstanz) 1-Chlorcarbonyl-3-methyl-2,4-dioxo-1,3-diazacyclohexan hergestellt.

Ausbeute: 1,0 Gew.-Teile

IR (Nujol; Carbonylbereich): 1770, 1722, 1700, 1662, 1602, 1530 (Schulter), 1520 (Schulter), 1503 $cm^{-1}$.

Schmp.: ab 198° dkl., ber. ca. 250° restgebende Zers.
Wirksamkeit (MHK in E/ml) bei E.coli Neumann = 8-16.

Beispiel 4

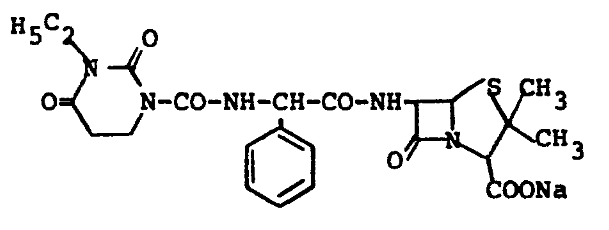

Dieses Penicillin wird in der in Beispiel 1 beschriebenen Weise aus 1,0 Gew.-Teilen α-Aminobenzylpenicillin (Ampicillin, Trihydrat) und 0,5 Gew.-Teilen (bezogen auf Reinsubstanz) 1-Chlorcarbonyl-3-äthyl-2,4-dioxo-1,3-diazacyclohexan hergestellt.

Ausbeute: 0,7 Gew.-Teile
Schmp.: ab ca. 195° Zers.

NMR (ppm, $CD_3OD$, 60 MHz), 1,15 (3 H, t, $CH_3-CH_2-$), 1,50 (3 H, s, $\times^{CH_3}_{CH_3}$ ), 1,54 (3 H, s, $\times^{CH_3}_{CH_3}$), 2,70 (2 H, t, $CH_2-CO-NEt$), ~3,66-4,05 (4 H, m, $N-CH_2-CH_3$ und $CH_2-N<^{CO}_{CO}$), 4,15 (1 H, s, 3-H), ~5,26-5,66 (3 H, m, $\underset{N}{-CH}-CO$ u. 5,6-H), 7,07-7,50 (5 H, m, Phenyl).

Die Substanz enthält nach dem NMR-Spektrum 0,09 Moläquivalente Natrium-2-äthylhexanoat und 1,55 Moläquivalente Wasser. Dieses wurde bei den folgenden berechneten Analysendaten berücksichtigt.

| | | | | | |
|---|---|---|---|---|---|
| Analyse: | ber. | C 48,9 | H 5,3 | N 12,0 | S 5,5 |
| | gef. | C 48,9 | H 5,3 | N 12,0 | S 5,5 |

| | | |
|---|---|---|
| Wirksamkeit (MHK in E/ml) | bei E. coli Neumann | = 4 |
| | bei Klebsiella 6097 | = 16 |
| | bei Proteus rettg. | = 16 |
| | bei Provid. 12012 | = 16 |

<u>Beispiel 5</u>

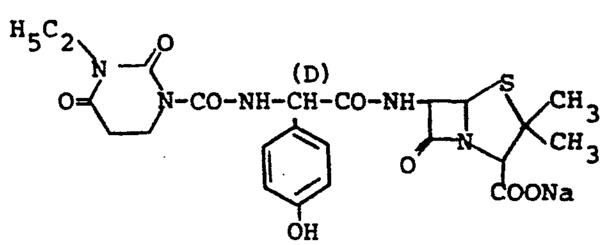

Dieses Penicillin wird in der im Beispiel 1 beschriebenen Weise aus 1,0 Gew.-Teilen α-Amino-p-hydroxybenzylpenicillin (Amoxycillin, Trihydrat) und 0,5 Gew.-Teilen (bezogen auf Reinsubstanz) 1-Chlorcarbonyl-3-äthyl-2,4-dioxo-1,3-diazacyclohexan hergestellt.

Ausbeute: 0,7 Gew.-Teile
Schmp.: ab ca. 220° rasche Zers.

NMR (ppm, $CD_3OD$) 1,14 (3 H, t, $N-CH_2-CH_3$), 1,50 (3 H, s, $\times^{CH_3}_{CH_3}$), 1,57 (3 H, s, $\times^{CH_3}_{CH_3}$ ), 2,68 (2 H, t, $CH_2-CO-NEt$), ~3,70-4,06 (4 H, m, $N-CH_2-CH_3$, $-CH_2-N<^{CO}_{CO}$), 4,13 (1 H, s, 3-H), ~5,26-5,53 (3 H, m, -CH(N)-CO, 5,6-H), 6,99 (4 H, AB, HO-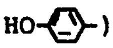-).

Wirksamkeit (MHK in E/ml) bei E. coli C 165 = 16
bei Prot. vulg. 9023 = 16

Beispiel 6

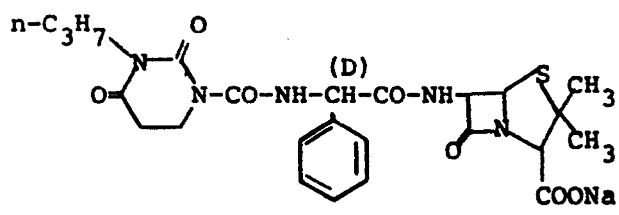

Dieses Penicillin wird in der im Beispiel 1 beschriebenen Weise aus 1,0 Gew.-Teilen α-Aminobenzylpenicillin (Ampicillin, Trihydrat) und 0,5 Gew.-Teilen (bezogen auf Reinsubstanz) 1-Chlorcarbonyl-3-n-propyl-2,4-dioxo-1,3-diazacyclohexan hergestellt.

Ausbeute: 0,6 Gew.-Teile
Schmp.: ab. ca. 206$^{o}$ rasche Zers.

NMR (ppm, $CD_3OD$, 60 MHz) 0,98 (3 H, t, n-Pr), ~1,18-1,93 (2 H, m, n-Pr), 1,56 (3 H, s, $\times^{CH_3}_{CH_3}$), 1,63 (3 H, s, $\times^{CH_3}_{CH_3}$), 2,80 (2 H, t, $CH_2$-CO-N-Pr), ~3,66-4,18 (4 H, m, n-Pr, $CH_2N{<}^{CO}_{CO}$), 4,24 (1 H, s, 3-H), 5,53 (2 H, AB, 5,6-H), 5,65 (1 H, s, $-\underset{\stackrel{|}{N}}{CH}-CO$), ~7,31 - 7,63 (5 H, m, Phenyl).

$C_{24}H_{28}N_5NaO_7S \times 2,2\ H_2O$

| | | | | |
|---|---|---|---|---|
| ber. | C 48,6 | H 5,5 | N 11,8 | S 5,4 |
| gef. | C 48,6 | H 5,4 | N 11,8 | S 5,5 |

Wirksamkeit (MHK in E/ml) bei E.coli 183/58 = 4
bei Klebs. 6097 = 16

Beispiel 7

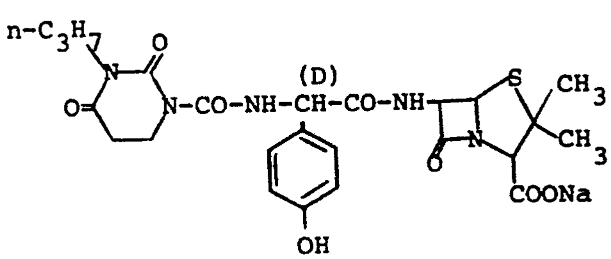

Dieses Penicillin wird erhalten, wenn man in der in Beispiel 1 beschriebenen Weise 1,0 Gew-Teile α-Amino-p-hydroxybenzylpenicillin (Amoxycillin, Trihydrat) und 0,5 Gew.-Teile (bezogen auf Reinsubstanz) 1-Chlorcarbonyl-3-n-propyl-2,4-dioxo-1,3-diazacyclohexan miteinander umsetzt.

Ausbeute: 0,8 Gew.-Teile
Schmp.: ab ca. 208° rasche Zers.

NMR (ppm, $CD_3OD$, 60 MHz), 0,90 (3 H, t, n-Pr), ~1,17-1,65 (2 H, m, n-Pr), 1,52 (3 H, s, $\times^{CH_3}_{CH_3}$), 1,58 (3 H, s, $\times^{CH_3}_{CH_3}$), 2,70 (2 H, t, $CH_2$-CO-N-Pr), ~3,37-4,09 (4H, m, N-Pr u. $CH_2-N<^{CO}_{CO}$), 4,14 (1 H, s, 3-H), 5,34-5,57 (3 H, m, $-\underset{N}{CH}-CO$ u. 5,6-H), 7,01 (4 H, AB, HO-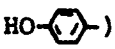-).

<u>Beispiel 8</u>

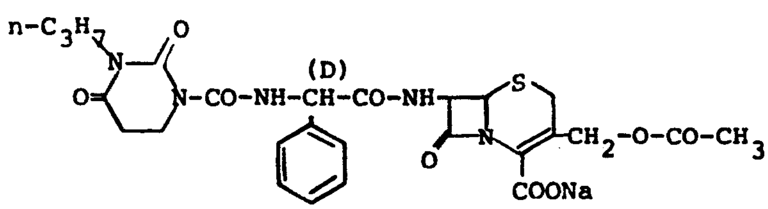

Dieses Cephalosporin wird erhalten, wenn man in der in Beispiel 1 beschriebenen Weise 1,0 Gew.-Teile α-Aminobenzylcephalosporin (Cephaloglycin, Dihydrat) und 0,5 Gew.-Teile (bezogen auf Reinsubstanz) 1-Chlorcarbonyl-3-n-propyl-2,4-dioxo-1,3-diazacyclohexan miteinander umsetzt.

Ausbeute: 0,6 Gew.-Teile
Schmp.: ab ca. 195° rasche Zers.

$C_{26}H_{28}N_5NaO_9S$ x 1,5 $H_2O$

ber. C 49,0 H 4,9 N 11,0 S 5,0
gef. C 48,9 H 4,9 N 11,0 S 5,0

Wirksamkeit (MHK in E/ml) bei E. coli Neumann = 4
bei Prot. vulg. 9023 = 8 - 16

Beispiel 9

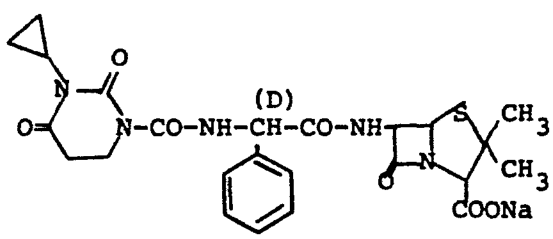

Dieses Penicillin wird erhalten, wenn man 1,0 Gew.-Teile α-Aminobenzylpenicillin (Ampicillin, Trihydrat) und 0,33 Gew.-Teile (bezogen auf Reinsubstanz) 1-Chlorcarbonyl-3-cyclopropyl-2,4-dioxo-1,3-diazacyclohexan miteinander umsetzt.

Ausbeute/ 0,65 Gew.-Teile
Schmp.: ab ca. 210°C rasche Zers.

NMR (ppm, $CD_3OD$, 60 MHz) ~ 0,83-1,38 (4 H, m, ), 1,50 (3 H, s, $CH_3$), 1,57 (3 H, s, $CH_3$), 2,68 (2 H, t, $-CH_2-CO-N$), ~3,13-3,73 (1 H, m, H), 3,92 (2 H, t, $CH_2-N\langle^{CO}_{CO}$), 5,46 (2 H, AB, 5,6-H), 5,56 (1 H, s, -CH-CO), 7,18 - 7,65 (5 H, m, Phenyl).

Die Substanz enthält nach dem NMR-Spektrum 0,21 Moläquivalente Natrium-2-äthylhexanoat und 1,55 Moläquivalente Wasser. Dieses wird bei der Berechnung der theoretischen Analysenwerte berücksichtigt:

| | | | | |
|---|---|---|---|---|
| ber. | C 50,2 | H 5,3 | N 11,4 | S 5,2 |
| gef. | C 50,2 | H 5,3 | N 11,4 | S 5,2 |

| | | |
|---|---|---|
| Wirksamkeit (MHK in E/ml) | bei Serratia 16 001 | = 16 |
| | bei Klebs. 63 | = 8 - 16 |
| | bei E. coli Neumann | = 4 |

<u>Beispiel 10</u>

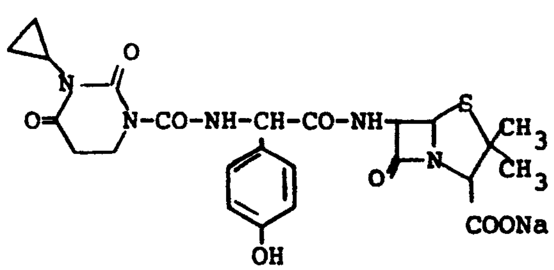

Dieses Penicillin wird erhalten, wenn man 1,0 Gew.-Teile α-Amino-p-hydroxybenzylpenicillin (Amoxycillin, Trihydrat) und 0,38 Gew.-Teile (bezogen auf Reinsubstanz) 1-Chlorcarbonyl-3-cyclopropyl-2,4-dioxo-1,3-diazacyclohexan miteinander umsetzt.

Ausbeute: 0,75 Gew.-Teile

Die Substanz enthält etwa 0,125 Moläquivalente Natrium-2-äthyl-hexanoat und 1,8 Moläquivalente Wasser. Dieses wurde bei den berechneten Werten der Analyse berücksichtigt:

| | | | | |
|---|---|---|---|---|
| ber. | C 48,3 | H 5,1 | N 11,3 | S 5,2 |
| gef. | C 48,3 | H 5,1 | N 11,4 | S 5,1 |

Wirksamkeit (MHK in E/ml) bei Psdm. F 41 = 64
bei E. coli 4322 = 16

Beispiel 11

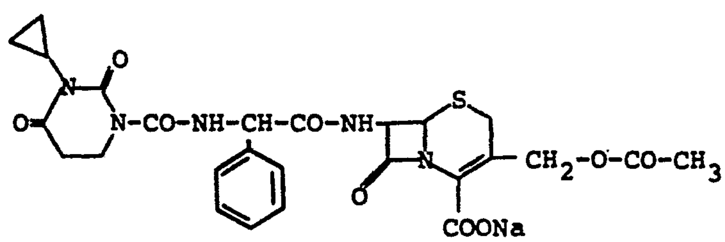

Dieses Cephalosporin wird inder im Beispiel 1 beschriebenen Herstellungweise erhalten, wenn man 1,0 Gew.-Teile α-Aminobenzylcephalosporin (Cephaloglycin, Dihydrat) und 0,33 Gew.-Teile (bezogen auf Reinsubstanz) 1-Chlorcarbonyl-3-cyclopropyl-2,4-dioxo-1,3-diazacyclohexan miteinander umsetzt.

Ausbeute: 0,62 Gew.-Teile

Schmp.: ab ca. 212° rasche Zers.

$C_{26}H_{26}N_5NaO_9S$ x 1,8 $H_2O$

| | | | | |
|---|---|---|---|---|
| ber. | C 48,8 | H 4,6 | N 10,9 | S 5,0 |
| gef. | C 48,5 | H 4,5 | N 10,9 | S 5,0 |

Wirksamkeit (MHK in E/ml) ber. E. coli 183/58 = 16

0004590



Patentansprüche

1) Verbindungen gemäß Formel I

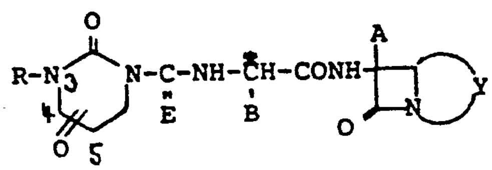

(I)

in der

R Alkyl, Alkenyl, Cycloalkyl, Cycloalkenyl, Cycloalkadienyl, wobei die genannten cyclischen Reste auch über eine Alkylengruppierung mit dem N-Atom verbunden sein können, Aralkyl, Aryl, Heterocyclyl, Heterocyclylalkyl, wobei die genannten Reste substituiert sein können oder eine Gruppe

$R_1$-CO- oder $R_2$-$SO_2$ ist,

in der

$R_1$,$R_2$ Alkyl, Alkenyl, Cycloalkyl, Cycloalkenyl, Cycloalkadienyl, wobei die genannten cyclischen Reste auch über eine Alkylengruppierung mit dem N-Atom verbunden sein können, Aralkyl, Aryl, Heterocyclyl, Heterocyclylalkyl, wobei die für $R_1$,$R_2$ vorstehend genannten Reste substituiert sein können; Amino, $R_3$-NH-, $(R_3)_2$-N- oder $R_3$-CH=N- bedeutet, wobei

$R_3$ ein Substituent aus der Reihe Alkyl, Alkenyl, Cycloalkyl, Cycloalkylalkyl, Aralkyl, Aryl, Heterocyclyl, Heterocyclylalkyl ist und

$R_1$ zusätzlich Wasserstoff oder $R_3$-O- mit der oben angegebenen Bedeutung für $R_3$ bezeichnet, steht;

die zweite Carbonylgruppe in 4- oder 5-Stellung steht, mit der Bedingung, daß das 3-ständige N-Atom nicht 3-fach acyliert ist;

E für Sauerstoff oder Schwefel steht;

B gegebenenfalls substituiertes Phenyl, einen gegebenenfalls substituierten heterocyclischen Rest, Cyclohexenyl oder Cyclohexadienyl bezeichnet;

A Wasserstoff, -OH-O-$R_4$ bedeutet, wobei

$R_4$ Alkyl, Alkenyl oder Cycloalkyl ist, wobei die für $R_4$ genannten Reste substituiert sein können,

Y für die Gruppen

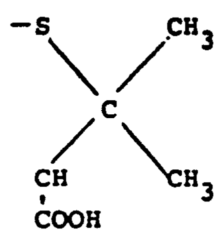

oder

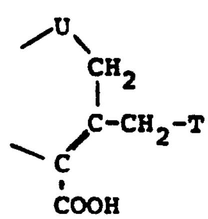

steht, in denen

U S, O oder SO bezeichnet,

T Wasserstoff, Alkyl-CO-O-, Pyridinium, Aminopyridinium, Carbamoylpyridinium, Carbamoyloxy, Azido, Cyano, Hydroxy, die Gruppe -S-Phenyl, welche substituiert sein kann, oder die Gruppe -S-Het bedeutet, in welcher Het für einen gegebenenfalls substituierten heterocyclischen 5- oder 6-gliedrigen Ring steht;

und in welchen das Kohlenstoffatom, welches die Carboxylgruppe trägt, an das Stickstoffatom des ß-Lactamringes gebunden ist und

wobei diese Verbindungen der Formel I bezüglich des Chiralitätszentrums $\overset{+}{C}$ in den beiden möglichen R- und S-Konfigurationen sowie als Gemische der daraus resultierenden Diastereomeren vorliegen können, sowie die Salze und Ester der Verbindungen gemäß Formel I.

2) Verbindungen gemäß Anspruch 1, bei denen E für Sauerstoff, A für Wasserstoff oder Methoxy, R, $R_1$, $R_2$ und $R_3$ für Alkyl mit 1 bis 6 C-Atomen, Alkenyl mit 2 bis 6 C-Atomen, Cycloalkyl, Cycloalkenyl, Cycloalkadienyl, Cycloalkylmethyl, Cycloalkenylmethyl, Cycloalkadienylmethyl, Cycloalkyläthyl, Cycloalkenyläthyl, Cycloalkadienyläthyl mit je 3 bis 10 C-Atomen im cyclischen Rest, Phenyl, Naphthyl, Benzyl, Phenyläthyl, einen 5- bis 7-gliedrigen heterocyclischen Rest, der auch über eine Methylen- oder Äthylenbrücke mit dem N-Atom des 1,3-Diazacyclohexansystems verbunden sein kann und wobei die genannten Reste substituiert sein können, stehen.

3) Verbindungen gemäß Anspruch 1, bei denen $R_1$ für Amino, $R_3$-NH, $(R_3)_2$-N-, $R_3$-CH=N-, $R_3$-O- und $R_2$ für Amino, $R_3$-NH oder $(R_3)_2$-N-, $R_3$-CH=N-, mit der in Anspruch 3 angegebenen Bedeutung für $R_3$ stehen.

4) Verbindungen gemäß Anspruch 1, bei denen Y für die Gruppe

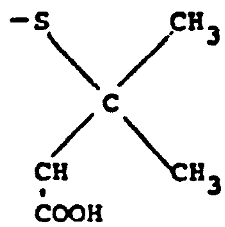

oder für die Gruppe

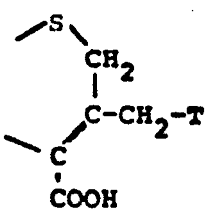

steht, wobei T für Wasserstoff, $CH_3$-COO-, Pyridinium, Aminopyridinium, Hydroxy oder einen heterocyclischen Rest der Formeln

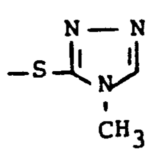 , 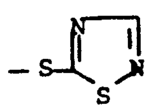 , 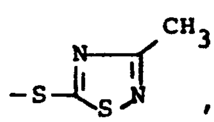 , 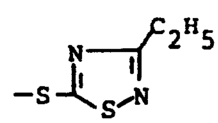 ,

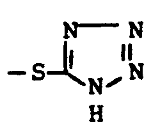 , 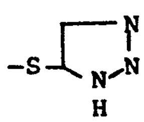 , 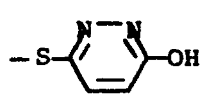 , 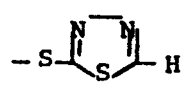 ,

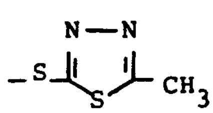 , 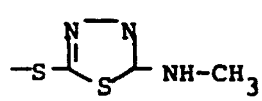 , 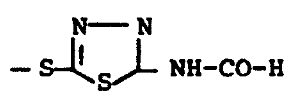 ,

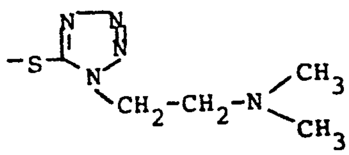 , 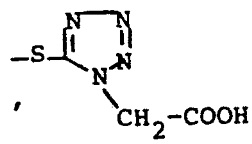 , 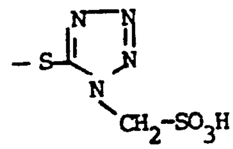 ,

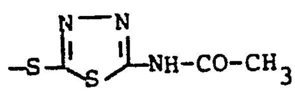 , 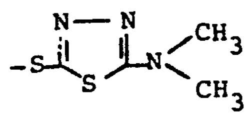 , 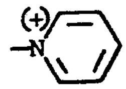 , 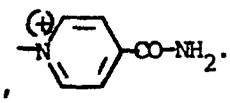

steht.

5) Verbindungen der Formel II

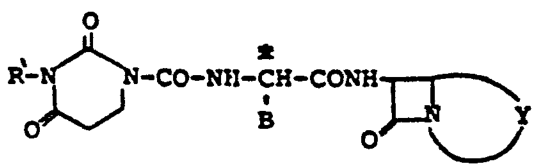 (II)

in der

R' für Alkyl mit 1 bis 6 Kohlenstoffatomen, Cycloalkyl mit 3 bis 6 Kohlenstoffatomen oder Phenyl steht,

B für Phenyl, p-Hydroxyphenyl, Thienyl-(2), 1,4-Cyclohexadien-(1)-yl steht und

Y eine Gruppe

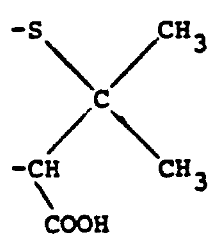 oder 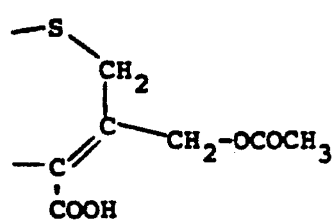 ,

bei der das C-Atom, welches die Carboxylgruppe trägt, an das N-Atom des ß-Lactamringes gebunden ist, bezeichnet.

6) Verfahren zur Herstellung von Verbindungen der Formel I

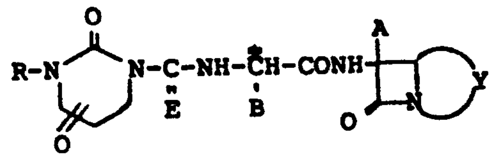

(I)

in der R, E, $\overset{*}{C}$, B, A und Y die in Anspruch 1 angegebene Bedeutung haben, dadurch gekennzeichnet, daß man Verbindungen der Formel III

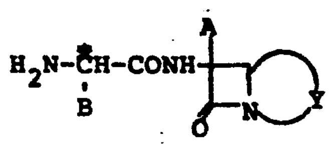

(III)

in der A, B, Y und $\overset{*}{C}$ die in Anspruch 1 angegebene Bedeutung besitzen, mit Verbindungen der Formel IV

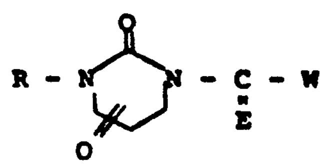

(IV)

in der R und E die oben angegebene Bedeutung besitzen,

W für Halogen, insbesondere Cl, Azid oder eine andere nukleofuge Abgangsgruppe steht,

oder wenn man Verbindungen der Formel V

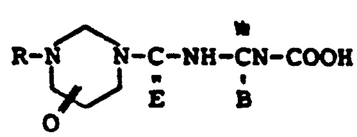

(V)

in einer an der Carboxylgruppe aktivierten Form, mit Verbindungen der Formel VI

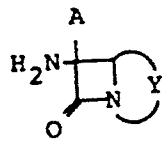

(VI)

in der

R und E die oben angegebene Bedeutung haben und

W für Halogen, insbesondere Cl, Azid oder eine andere nukleofuge Abgangsgruppe steht,

in Gegenwart eines Lösungsmittels und gegebenenfalls eines Säurebindemittels bei Temperaturen von etwa -20 bis etwa +50°C umsetzt, gegebenenfalls Aminoschutzgruppen entfernt und die erhaltenen ß-Lactam-Antibiotica gegebenenfalls in Salze und Ester überführt oder aus den erhaltenen Salzen gewünschtenfalls die freien Säuren herstellt.

Ferner können die ß-Lactam-Verbindungen der Formel I die anstelle von A den Rest $-OR_2$ tragen, dadurch hergestellt werden, daß man die Verbindungen der Formel I, die anstelle von A Wasserstoff tragen und in denen R, $R_2$, E, B und Y die oben angegebene Bedeutung haben, mit 2 bis 10 Äquivalenten einer Base in Anwesenheit eines Überschusses eines Alkohols der Formel $R_2OH$, in der $R_2$ die oben angegebene Bedeutung besitzt, in einem inerten organischen Lösungsmittel umsetzt, zwischen 1 bis 8 Äquivalente eines N-Halogenierungsmittels zufügt und die Verbindung der Formel (I), gegebenenfalls nach vorheriger Abspaltung der Säureschutzgruppe und/oder Überführung in ein Salz, isoliert; in Gegenwart eines Lösungsmittels und gegebenenfalls eines Säurebindemittels bei Temperaturen von etwa -20°C bis etwa +50°C umsetzt und die erhaltenen ß-Lactam-Antibiotica gegebenenfalls in Salze und Ester überführt oder aus den erhaltenen Salzen gewünschtenfalls die freien Säuren herstellt.

7) Arzneimittel, gekennzeichnet durch einen Gehalt an einer Verbindung gemäß den Ansprüchen 1 bis 5.

8) Verfahren zur Behandlung von durch Bakterien hervorgerufenen Erkrankungen, dadurch gekennzeichnet, daß man Verbindungen gemäß den Ansprüchen 1 bis 5 Menschen oder Tieren appliziert, die an diesen Erkrankungen leiden.

9) Futterzusatzmittel, gekennzeichnet durch einen Gehalt an einer Verbindung gemäß den Ansprüchen 1 bis 5.

10) Verwendung von Verbindungen gemäß den Ansprüchen 1 bis 5 zur Förderung des Wachstums und zur Verbesserung der Futterverwertung bei Tieren.

0004590

Europäisches Patentamt

# EUROPÄISCHER RECHERCHENBERICHT

Nummer der Anmeldung
EP 79 10 0826

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 2 519 400 (TOYAMA)<br>* Seiten 222-236; Ansprüche 1,10, 11,48 *<br>---- | 1,6-8 |

KLASSIFIKATION DER ANMELDUNG (Int.Cl.²)

C 07 D 501/20
499/64
A 61 K 31/545
31/43
A 23 K 1/17

RECHERCHIERTE SACHGEBIETE (Int. Cl.²)

C 07 D 501/20
499/64

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

√ Der vorliegende Recherchenbericht wurde für alle Patentansprüche erstellt.

| Recherchenort | Abschlußdatum der Recherche | Prüfer |
|---|---|---|
| Den Haag | 15-06-1979 | LUYTEN |